**Europäisches Patentamt**

**European Patent Office**

**Office européen des brevets**

(19)

(11) Veröffentlichungsnummer : **0 318 801 B1**

(12) **EUROPÄISCHE PATENTSCHRIFT**

(45) Veröffentlichungstag der Patentschrift :
25.09.91 Patentblatt 91/39

(51) Int. Cl.⁵ : **A61K 47/00**

(21) Anmeldenummer : 88119390.8

(22) Anmeldetag : 22.11.88

(54) **Zubereitungsformen zur Verhinderung von Adhäsionen von Organen und Organteilen.**

(30) Priorität : 04.12.87 DE 3741149

(43) Veröffentlichungstag der Anmeldung :
07.06.89 Patentblatt 89/23

(45) Bekanntmachung des Hinweises auf die
Patenterteilung :
25.09.91 Patentblatt 91/39

(84) Benannte Vertragsstaaten :
AT BE CH DE ES FR GB GR IT LI LU NL SE

(56) Entgegenhaltungen :
EP-A- 0 227 400
WO-A-84/00111

(73) Patentinhaber : **Dr. Karl Thomae GmbH**
**Postfach 1755**
**W-7950 Biberach 1 (DE)**

(72) Erfinder : **Franz, Helmut, Dr.**
**Obere Au 2**
**W-7950 Biberach 1 (DE)**
Erfinder : **Müller, Thomas, Dr. Dipl.-Chem.**
**Gymnasiumstrasse 16**
**W-7950 Biberach 1 (DE)**
Erfinder : **Eisert, Wolfgang, Prof. Dr. Dr. Dr.**
**Friedrich-Goll-Weg 5**
**W-7950 Biberach 1 (DE)**

**Beschreibung**

Die Erfindung betrifft neue Zubereitungsformen zur Verhinderung von Adhäsionen von Organen und- /oder Organteilen.

Nach invasiven therapeutischen Eingriffen oder im Verlauf einer Erkrankung können Verklebungen von Organen oder Organteilen zu lebensbedrohlichen Situationen führen. Die Bildung von Adhäsionen wird auch nach chirurgischen Eingriffen in der Brusthöhle oder in der Bauchhöhle beobachtet. Trotz erhöhter Anstrengungen, solche Adhäsionen zu verhindern, wurde bis heute keine zufriedenstellende Behandlungsmethode gefunden.

Die Rolle der Fibrinbildung bei der Adhäsionsbildung läßt sich wie folgt erklären : nach invasiven therapeutischen Eingriffen oder bei entzündlichen Reaktionen kommt es zum Austritt von Plasmaproteinen, wie auch von Fibrinogen und anderen Gerinnungsproteinen aus dem Gewebe. Das Fibrinogen schlägt sich als Fibrin nieder. Das sich ausbildende Fibrinnetzwerk verbindet (verklebt) dann benachbarte Oberflächen von Organen oder anderen Organteilen. Wird das Fibrin nicht aufgelöst, so bilden sich dichte Adhäsionen, die zum Beispiel zu gefährlichen Darmverschlingungen führen können. Fibrinverklebungen, die frisch gebildet worden sind, werden nach und nach durch Fibroblasten zu festen Gewebeverbindungen umgebaut.

Der Grad einer möglichen spontanen Fibrinolyse hängt von der Freigabe des Gewebeplasminogenaktivators (t-PA) aus dem vasculären Endothelium aber auch aus mesothelialen Zellen, wie sie im Bauchraum vorliegen, ab. Nach einem peritonealen Eingriff kommt es aber zu einer Reduktion der fibrinolytischen Aktivität in diesen mesothelialen Zellen. Wird dadurch die Fibrinolyse unvollständig, so verhalten sich die Fibrinreste als Zentren, in welche Fibroblasten einwachsen ; es bilden sich Kapillaren, die eine Fibrinadhäsion verursachen. Diese werden später durch Collagen enthaltende Adhäsionen ersetzt, wobei das Collagen durch die Fibroblasten synthetisiert wird.

Zur Verhinderung der Bildung postoperativer oder entzündungsbedingter Adhäsionen wurde eine systemische Verabreichung von Ibuprofen vorgeschlagen (U.S. Patent 4.346.108) ; andere Vorschläge betreffen die parenterale Verabreichung von Antihistaminen, Corticosteroiden und Antibiotika oder die intraperitoneale Verabreichung von Dextranlösungen oder Polyvinylpyrrolidonlösungen. Auch die einschlägige Anwendung von Streptokinase, Streptodornase und Urokinase wurde vorgeschlagen [vgl. Ascherl et al., Medwelt 34, No. 13/83, Seiten 410-415; Mund-Hoym et al., Geburtsb. u. Frauenheilkunde 44 (1984), Seiten 463-467 ; Minju et al., Acta Academiae Medicinae Wuhan 3, (2), Seiten 77-83].

Auch Human-Fibrinolysin wurde allein oder in Verbindung mit anderen Medikamenten auf seine Fähigkeit, postoperative Adhäsionen zu unterbinden, untersucht [vgl. Gazzaniga et al., Arch. Surg. Vol. 110, Seiten 429-432 (1975). Holtz, The Journal of Reproductive Medicine, Vol. 24, No. 4 (1980), Seiten 141-146, Rivkind et al., Eur. Surg. Res. (Schweiz) 1985, Vol. 17, Nr. 4, Seiten 254-258 und Buckman et al., Journal of Surgical Research, Vol. 20, Nr. 1, Seiten 1-5] kamen zu dem Schluß, daß die postoperative Bildung von Adhäsionen in der Bauchhöhle mit einer traumatisch oder ischämisch induzierten Reduktion der Aktivität des Plasminogenaktivators verbunden ist.

Die daraus zu folgernde direkte topische Anwendung des Gewebsplasminogenaktivators (t-PA) zur Verhinderung postoperativer intraperitonealer Adhäsionen wurde schließlich in der EP-A-0227400 beschrieben. T-PA, welches im Körper sowohl als einsträngiges als auch als doppelsträngiges Molekül vorkommt, besitzt eine hohe Affinität für Fibrin, welches die Thromben aufbaut. Das natürliche t-PA ist glycosiliert und enthält Fettsäurereste. Alle diese Arten des t-PA besitzen eine spezifische Affinität für Fibrin und aktivieren gleichzeitig das Plasminogen zu Plasmin. Plasmin bewirkt hierbei den proteolytischen Abbau des Fibrins. Da das Fibrin pathophysiologisch für die Bildung von Adhäsionen verantwortlich ist, führt t-PA zu einer Verhinderung der Adhäsionsbildung und, gegebenenfalls, auch zur Auflösung bereits gebildeter Verklebungen, z.B. im Bauchraum nach operativen Eingriffen oder nach entzündlichen Prozessen. Das hierbei verwendete t-PA wird aus menschlichem Gewebe isoliert oder unter Zuhilfenahme der rekombinanten DNA-Technologie (vgl. GB-A-2.119.804, EP-A-0174835 und 0100982) gewonnen.

Gemäß der obengenannten EP-A-0227400 wird t-PA topisch z.B. im Bereich der chirurgischen Einwirkung möglichst gleich nach Beendigung derselben bzw. vor dem Einsetzen der Wundheilung zur Verhinderung des Verklebens von Gewebs- und/oder Organteilen oder bei aufkommenden entzündlichen Prozessen auf die entsprechenden Bereiche aufgetragen, eventuell ergänzend wird auch eine t-PA-Zubereitung über einen am Orte des Eingriffs endenden Katheter langsam appliziert.

Beschrieben werden sterile t-PA-Zubereitungsformen, die einen pharmazeutisch verträglichen Träger enthalten, z.B. eine phosphatgepufferte Kochsalzlösung, eine isotonische Kochsalzlösung oder gereinigtes Wasser. Als organische Träger kommen Lipide, z.B. Phosphorlipidmicellen oder -vesikel, aber auch Dextran, Polymere, wie p-Dioxanone, Lactide und/oder Glycolide in Form von adsorbierbaren Polymeren, die mikroverkapselt oder in Salbengrundlagen eingebettet sind oder in einer wäßrigen Lösung eines oberflächenaktiven Stoffes, z.B. eines Polyoxyethylen-polyoxypropylen-block-copolymers oder eines Sorbitanfettsäureester-polyoxyethylenethers, vorliegen, in Frage. Als bevorzugte Zubereitungsformen werden solche angesehen, bei welchen t-PA in einem Träger mit verzögerter Wirkstoff-Freigabe enthalten ist, der den Wirkstoff gesteuert innerhalb von einem bis 7 Tage freigibt. Als Träger mit verzögerter kontrollierter Wirkstoff-Freigabe werden adsorbierbare Polymere, die als Mikrokapseln oder in einer Salbengrundlage vorliegen, insbesondere aber Phospholipid-Vesikel, sogenannte Liposome, genannt.

In der WO-A-8400111 werden Zubereitungsformen zur Behandlung von Verbrennungen, Schnitten, Wunden und Abschürfungen der äußeren Körperschichten (Haut) beschrieben. Diese Zubereitungsformen bestehen aus einem Hitze-sterilidierbaren wässerigen Gel, das ein pharmazeutisch anwendbares Glycol und ein Hitze-sterilisierbares Cellulosederivat, z.B. Hydroxyethylcellulose, und, gegebenenfalls, ein Antiseptikum oder Antibiotikum enthält.

Es wurde nun gefunden, daß t-PA oder rt-PA (r = rekombinant), gelöst in einem Hydroxyethylcellulosegel, die Bildung von Adhäsionen nahezu vollständig verhindert, auch wenn dieses Hydrogel nur einmalig, d.h. kurz nach dem chirurgischen Eingriff oder nach einem entzündlichen Reiz auf die zu Verklebungen und Verwachsungen neigenden Zonen aufgetragen wird.

Zur Herstellung einer geeigneten Zubereitungsform wird eine mikrobiologisch reine Hydroxyethylcellulose in Wasser zu einem Hydrogel gelöst. Dem Wasser können zur Isotonisierung Salze, z.B. Natriumchlorid, aber auch Puffersubstanzen, wie Dikaliumhydrogenphosphat und Natriumdihydrogenphosphat, beigefügt sein. Das vorzugsweise lyophilisierte t-PA oder rt-PA wird in dem sterilisierten Hydrogel gelöst, wobei dieses gebrauchsfertige Hydrogel auch für einige Tage bei niederen Temperaturen gelagert werden kann. Für längere Lagerzeiten ist es aber von Vorteil, das sterilisierte Hydrogel und das lyophilisierte t-PA oder rt-PA in getrennten Behältnissen aufzubewahren und die fertige Lösung erst kurz vor ihrer Anwendung durch Mischung dieser beiden Komponenten herzustellen.

Untersuchungen ergaben, daß auch das Hydroxyethylcellulose-Hydrogel selbst eine adhäsionsverhindernde Wirkung ausübt, die bis zu 40% im Vergleich zu Fällen, bei welchen keine derartige Prophylaxe durchgeführt wurde, ausmacht. Das Hydroxyethylcellulose-Hydrogel erfährt durch die Anwesenheit von t-PA eine Steigerung seiner prophylaktischen Aktivität.

Eine erfindungsgemäße Hydrogelzubereitung läßt sich auch, bei reduzierter Viskosität, leicht, z.B. durch einen Kanal an einem Endoscop, an gefährdete Stellen, z.B. in Körperhöhlen, instillieren.

Ein erfindungsgemäßes wäßriges Hydrogel besteht aus 1 bis 3 g Hydroxyethylcellulose, gelöst in 97 bis 99 g Wasser oder physiologischer Kochsalzlösung, und enthält 0,05 bis 50 mg/ml t-PA oder rt-PA, vorzugsweise aber 0,3 bis 10 mg/ml mit oder ohne Zusatz von basischen Aminosäuren, z.B. Lysin, Argenin.

Als wasserlösliche Hydroxyethylether der Cellulose dienen vorzugsweise solche mit einem mittleren molekularen Substitutionsgrad von 1,5 bis 3,0 Hydroxyethylgruppen pro Anhydroglucoseeinheit. Ein bevorzugtes Hydrogel besteht aus 2 Gew.-% Hydroxyethylcellulose (z.B. Natrosol 250 HX(R)), gelöst in Wasser, physiologischer Kochsalz- oder Pufferlösung und enthält 2 mg/ml t-PA oder rt-PA.

Eine Mischung von 1 mg/ml rt-PA und 2 Gew.-% Hydroxyethylcellulose in physiologischer Kochsalzlösung wurde vergleichend mit einer Mischung aus 2 Gew.-% Hydroxyethylcellulose in physiologischer Kochsalzlösung und Kontrollen (nur physiologische Kochsalz- bzw. Pufferlösung) an Kaninchen untersucht. Hierbei wurden Adhäsionen erzeugt

a) durch Anbringen einer Naht in der Bauchwandung nach Laparotomie und

b) durch Reizung des peritonealen Gewebes im Bereich von Zoekum und Ileum und des kleinen Beckens mit einer Jodlösung.

Bei der Anbringung einer Naht in der Bauchwandung nach Laparotomie (Fall a) wurde das Testmaterial aufgebracht und die Laparotomienaht mehrschichtig verschlossen. Dem Operateur bzw. dem Untersucher war die Zusammensetzung des Testmaterials nicht bekannt. Nach einer Woche wurde in einem großen Hautschnitt das Gebiet der alten Laparotomienaht umschnitten und aufgeklappt, es wurde die Länge des adhärierenden Darmgewebes an der Laparotomienaht gemessen und die verwachsene Länge als Prozent der Laparotomienahtlänge ausgedrückt.

Das Testmaterial (mit und ohne t-PA) wurde im Fall b) sofort nach dem Eingriff und vor dem Wiederverschließen der Bauchhöhle appliziert. Auch hierbei war dem Operateur die Zusammensetzung des Testmaterials nicht bekannt.

Sieben Tage nach diesem Eingriff wurden die Tiere in Narkose relaparotomiert, um das Ausmaß der Verklebungen zu ermitteln. Dazu wurde die Fläche zugfester Verklebungen der behandelten Darmabschnitte in den geschädigten Bereichen gemessen. Die Ergebnisse sind als prozentuale Verklebung bezogen auf die Fläche der geschädigten Bereiche dargestellt. Zusätzlich wurde das Körpergewicht der Tiere am Tag der Erst- und der Relaparotomie quantifiziert.

Die Ergebnisse dieser Versuche sind in der Tabelle 1 dargestellt. In der Abzisse ist das Ausmaß der prozentualen Verklebung bzw. der prozentualen Veränderung des Körpergewichts dargestellt für

1.) Kontrolle,

2.) Natrosol (HEC) 2 Gew.-% in physiologischer Kochsalzlösung und

3.) t-PA in Natrosol (1 mg rt-PA pro ml physiologischer Kochsalzlösung mit 2 Gew.-% HEC).

Ein Vergleich der Ergebnisse der Tabelle 1 zeigt, daß das Hydroxyethylcellulose-Hydrogel allein am Zoekum und Ileum im Mittel eine 40 bis 45%ige Reduktion der durch die Bedingungen induzierten Verklebungen (bezogen auf die Kontrollen) verursachte, in Verbindung mit rt-PA aber am Zoekum eine nahezu vollständige (95%ige) Reduktion, am Ileum eine ca. 85%ige Reduktion herbeiführte. Für die Verklebung der Laparotomienaht (Bauchwand) mit den darunterliegenden Geweben bewirkt das Hydrogel allein bereits eine beträchtliche Reduktion ; durch die Gegenwart von rt-PA wird die Verklebung aber vollständig unterdrückt.

Durch die Anwendung der erfindungsgemäßen, t-PA und rt-PA enthaltenden Hydrogelzubereitungen wird der Verlust an Körpergewicht während der ersten Heilungsphase signifikant minimiert.

Die folgenden Beispiele beschreiben die Herstellung von erfindungsgemäßen Zubereitungsformen.

Beispiel 1

rt-PA-set mit 0,1 mg rt-PA/ml

1.) In einem geeigneten Behältnis wird in 98 g sterilfiltrierte physiologische Kochsalzlösung 2 g mikrobiologisch reine Hydroxyethylcellulose eingestreut und unter Rühren bei Raumtemperatur in Lösung gebracht. Das gebildete Gel wird unter Laminar-air-flow in 50 ml-Steckkappenflaschen abgefüllt, mit Gummistopfen verschlossen und gebördelt sowie anschließend 30 Minuten bei 121°C im gespannten Wasserdampf sterilisiert. Das verschlossene Gel ist bei Raumtemperatur zwei Jahre lagerfähig.

2.) rt-PA wird zu 10, 20 und 30 mg unter sterilen Bedingungen in Steckkappenfläschchen lyophilisiert, mit Gummistopfen verschlossen und gebördelt. Das verschlossene Fläschchen ist bis zu zwei Jahre bei Raumtemperatur (bis zu 25°C) lagerfähig.

3.) Gebrauchsfertige Lösung :

In 50 g sterilem 2 Gew.-%igem Hydroxyethylcellulosegel werden unter Laminar-air-flow-Bedingungen 5 mg lyophilisiertes rt-PA eingebracht und mit einem sterilen Glasstab unter Rühren gelöst.

Dieses gebrauchsfertige Gel ist entweder sofort auf die Wundnaht aufzubringen oder bis zu einigen Tagen im Kühlschrank bei 4°C lagerfähig.

Beispiel 2

rt-PA-Gel mit 1,0 mg rt-PA/ml

Vor Gebrauch werden in 50 g sterilem und isotonisiertem 2 Gew.-%igem Hydroxyethylcellulosegel 50 mg lyophilisiertes rt-PA eingebracht und mit einem sterilen Glasstab unter Rühren gelöst.

Das gebrauchsfertige Gel ist bis zu einigen Tagen im Kühlschrank lagerfähig.

Beispiel 3

rt-PA-Gel mit 10,0 mg rt-PA/ml

Vor Gebrauch werden in 50 g sterilem und isotonisiertem 2 Gew.-%igem Hydroxyethylcellulosegel 500 mg lyophilisiertes rt-PA eingebracht.

Das gebrauchsfertige Gel ist bis zu einigen Tagen im Kühlschrank lagerfähig.

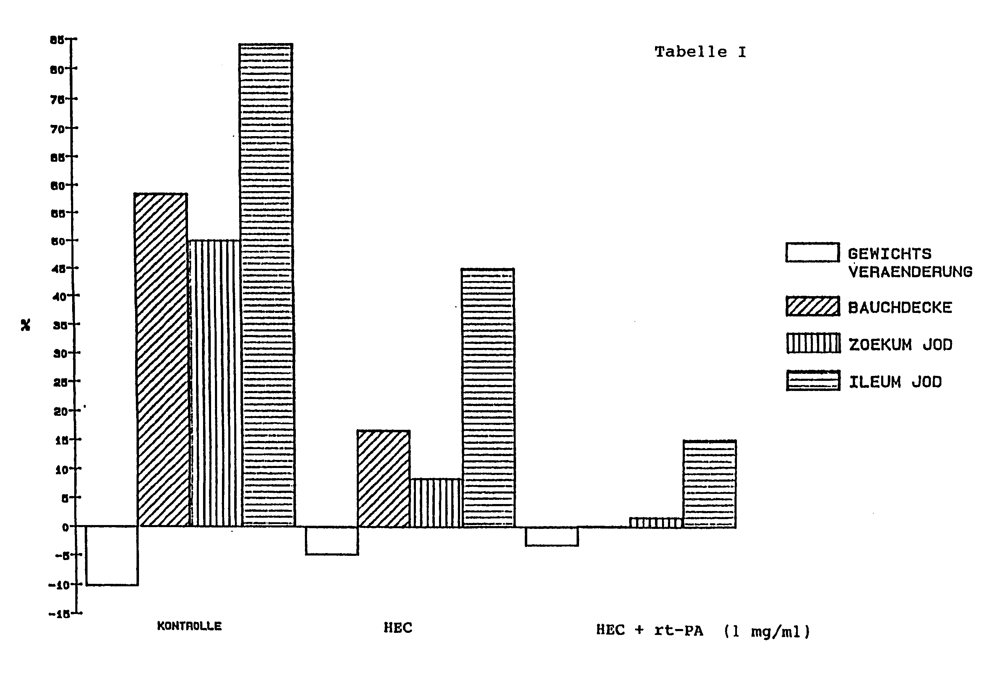

**Patentansprüche**

**Patentansprüche für folgenden Vertragsstaaten : AT, BE, CH, DE, FR, GB, IT, LI, LU, NL, SE**

1. Zubereitungsformen zur Verhinderung von Adhäsionen von Organen und Organteilen nach invasiven therapeutischen Eingriffen oder im Verlauf einer Erkrankung enthaltend ein wäßriges Hydrogel auf Basis Hydroxyethylcellulose und t-PA oder rt-PA.

2. Zubereitungsformen gemäß Anspruch 1, dadurch gekennzeichnet, daß dem t-PA oder rt-PA enthaltenden Hydroxyethylcellulose-Hydrogel noch isotonisierende Zusätze und gegebenenfalls Puffersubstanzen beigefügt sind.

3. Zubereitungsformen gemäß Anspruch 1 und 2, dadurch gekennzeichnet, daß diese als Hydrogel 1 bis 3 Gew.-% Hydroxyethylcellulose in wäßriger Lösung und 0,05 bis 50 mg/ml t-PA oder rt-PA enthalten.

4. Zubereitungsform gemäß Anspruch 3, dadurch gekennzeichnet, daß diese 0,3 bis 10 mg/ml t-PA oder rt-PA enthält.

5. Zubereitungsformen gemäß Anspruch 1, 2, 3 und 4, dadurch gekennzeichnet, daß diese als Hydroxyethylether der Cellulose einen solchen mit einem mittleren molekularen Substitutionsgrad von 1,5 bis 3,0 Hydroxyethylgruppen pro Anhydroglucoseeinheit enthalten.

6. Zubereitungsform gemäß Anspruch 1 bis 5, dadurch gekennzeichnet, daß diese 2 Gew.-% Hydroxyethylcellulose und 1 bis 2 mg/ml rt-PA in einer Pufferlösung enthält.

7. Zubereitungsformen gemäß Anspruch 1 bis 6, dadurch gekennzeichnet, daß das sterilisierte Hydrogel und das lyophilisierte t-PA oder rt-PA in getrennten Behältnissen gelagert werden, die es gestatten, die fertige Lösung erst kurz vor ihrer Anwendung durch Mischung dieser beiden Komponenten herzustellen.

8. Verwendung von Hydroxyethylcellulose und t-PA oder rt-PA in wäßriger Lösung zur Herstellung von Zubereitungsformen zur Verhinderung von Adhäsionen von Organen oder Organteilen nach invasiven Eingriffen oder im Verlauf einer Erkrankung gemäß den Ansprüchen 1 bis 7.

**Patentansprüche für folgende Vertragsstaaten : ES, GR**

1. Verfahren zur Herstellung von Zubereitungsformen zur Verhinderung von Adhäsionen von Organen und Organteilen nach invasiven therapeutischen Eingriffen oder im Verlauf einer Erkrankung dadurch gekennzeichnet, daß eine sterilisierte Hydroxyethylcellulose in Wasser zu einem Hydrogel gelöst und t-PA oder rt-PA in diesem Hydrogel zur Lösung gebracht wird.

2. Verfahren gemäß Anspruch 1, dadurch gekennzeichnet, daß dem Wasser zur Isotonisierung Salze und, gegebenenfalls, auch Puffersubstanzen beigefügt werden.

3. Verfahren gemäß Anspruch 1 und 2, dadurch gekennzeichnet, daß 1 bis 3 g Hydroxyethylcellulose in 97 bis 99 g Wasser oder physiologischer Kochsalzlösung gelöst werden und in der Lösung 0,05 bis 50 mg/ml t-PA oder rt-PA aufgelöst werden.

4. Verfahren gemäß Anspruch 3, dadurch gekennzeichnet, daß eine Hydroxyethylcellulose mit einem mittleren molekularen Substitutionsgrad von 1,5 bis 3 Hydroxyethylgruppen pro Anhydroglucoseeinheit zur Herstellung des Hydrogels verwendet wird.

**Claims**

**Claims for the following Contracting States : AT, BE, CH, DE, FR, GB, IT, LI, LU, NL, SE**

1. Preparations for preventing adhesions of organs and parts of organs after invasive therapeutic intervention or in the course of a disease, containing an aqueous hydrogel based on hydroxyethylcellulose and t-PA or rt-PA.

2. Preparations according to claim 1, characterised in that isotonic additives and optionally buffer substances are also added to the hydroxyethylcellulose hydrogel containing the t-PA or rt-PA.

3. Preparations according to claim 1 and 2, characterised in that they contain as hydrogel 1 to 3% by weight of hydroxyethylcellulose in aqueous solution and 0.1 to 50 mg/ml of t-PA or rt-PA.

4. Preparation according to claim 3, characterised in that it contains 0.3 to 10 mg/ml of t-PA or rt-PA.

5. Preparations according to claims 1, 2, 3 and 4, characterised in that they contain as hydroxyethylethers of cellulose those having an average molecular substitution level of from 1.5 to 3.0 hydroxyethyl groups per unit of anhydroglucose.

6. Preparation according to claims 1 to 5, characterised in that it contains 2% by weight of hydroxyethylcellulose and 1 to 2 mg/ml of rt-PA in a buffer solution.

7. Preparations according to claims 1 to 6, characterised in that the sterilised hydrogel and the lyophilised t-PA or rt-PA are stored in separate containers which enable the finished solution to be made up just before use by mixing these two components.

8. Use of hydroxyethylcellulose and t-PA or rt-PA in aqueous solution for the production of preparations for preventing adhesions of organs or parts of organs after invasive interventions or in the course of a disease according to claims 1 to 7.

**Claims for the following Contracting States : ES, GR**

1. Process for producing preparations for preventing adhesions of organs and parts of organs after invasive therapeutic intervention or in the course of a disease, characterised in that a sterilised hydroxyethylcellulose is dissolved in water to form a hydrogel and t-PA or rt-PA is dissolved in this hydrogel.

2. Process according to claim 1, characterised in that salts are added to the water to render it isotonic and optionally buffer substances are also added.

3. Process according to claims 1 and 2, characterised in that 1 to 3 g of hydroxyethylcellulose are dissolved in 97 to 99 g of water or physiological saline solution and 0.05 to 50 mg/ml of t-PA or rt-PA are dissolved in the solution.

4. Process according to claim 3, characterised in that a hydroxyethylcellulose with an average molecular substitution level of from 1.5 to 3 hydroxyethyl groups per unit of anhydroglucose is used to prepare the hydrogel.

**Revendications**

**Revendications pour les Etats Contractants suivants : AT, BE, CH, DE, FR, GB, IT, LI, LU, NL, SE**

1. Compositions pour prévenir les adhésions d'organes et de parties d'organes à la suite d'interventions thérapeutiques invasives ou au cours d'une maladie, contenant un hydrogel aqueux à base d'hydroxyéthylcellulose et de t-PA ou de rt-PA.

2. Compositions selon la revendication 1, caractérisées en ce qu'à l'hydrogel d'hydroxyéthylcellulose contenant du t-PA ou du rt-PA sont ajoutés encore des additifs pour le rendre isotonique et éventuellement des substances tampons.

3. Compositions selon les revendications 1 et 2, caractérisées en ce qu'elles contiennent comme hydrogel 1 à 3% en poids d'hydroxyéthylcellulose en solution aqueuse et 0,05 à 50 mg/ml de t-PA ou de rt-PA.

4. Composition selon la revendication 3, caractérisée en ce qu'elle contient 0,3 à 10 mg/ml de t-PA ou de rt-PA.

5. Compositions selon les revendications 1, 2, 3 et 4, caractérisées en ce qu'elles contiennent comme hydroxyéthyléther de cellulose un hydroxyéthyléther ayant un degré de substitution moléculaire moyen de 1,5 à 3,0 groupes hydroxyéthyle par unité d'anhydroglucose.

6. Composition selon les revendications 1 à 5, caractérisée en ce qu'elle contient 2% en poids d'hydroxyéthylcellulose et 1 à 2 mg/ml de rt-PA dans une solution tampon.

7. Compositions selon les revendications 1 à 6, caractérisées en ce que l'hydrogel stérilisé et le t-PA ou rt-PA lyophilisé sont conservés dans des récipients séparés qui permettent de préparer la solution terminée peu de temps avant son utilisation par mélange de ces deux constituants.

8. Utilisation de l'hydroxyéthylcellulose et du t-PA ou du rt-PA en solution aqueuse pour la préparation de compositions pour prévenir les adhésions d'organes ou de parties d'organes à la suite d'interventions invasives ou au cours d'une maladie, selon les revendications 1 à 7.

**Revendications pour les Etats Contractants suivants : ES, GR**

1. Procédé de préparation de compositions pour prévenir les adhésions d'organes et de parties d'organes à la suite d'interventions thérapeutiques invasives ou au cours d'une maladie, caractérisé en ce qu'une hydroxyéthylcellulose stérilisée est dissoute dans l'eau en un hydrogel et du t-PA ou du rt-PA est mis en solution dans cet hydrogel.

2. Procédé selon la revendications 1, caractérisé en ce que des sels pour rendre isotonique et éventuellement également des substances tampons sont ajoutés à l'eau.

3. Procédé selon les revendications 1 et 2, caractérisé en ce que 1 à 3 g d'hydroxyéthylcellulose sont dissous dans 97 à 99 g d'eau ou de sérum physiologique et 0,05 à 50 mg/ml de t-PA ou de rt-PA sont dissous dans la solution.

4. Procédé selon la revendication 3, caractérisé en ce que l'on utilise une hydroxyéthylcellulose ayant un degré de substitution moléculaire moyen de 1,5 à 3 groupes hydroxyéthyle par unité d'anhydroglucose pour la préparation de l'hydrogel.

Tabelle I

GEWICHTS VERAENDERUNG
BAUCHDECKE
ZOEKUM JOD
ILEUM JOD

KONTROLLE          HEC          HEC + rt-PA   (1 mg/ml)